# EUROPEAN PATENT SPECIFICATION

(11) **EP 0 589 375 B1**
(45) Date of publication and mention of the grant of the patent: **02.04.1997**
(21) Application number: 93115055.1
(22) Date of filing: 18.09.1993
(51) Int. Cl.: A61K 9/14

(54) **Micronutrient compositions**
Mikronährstoff-Zusammensetzungen
Compositions de substances micronutritives

(30) Priority: 23.09.1992 US 950381
(43) Date of publication of application: 30.03.1994
(73) Proprietor: F. HOFFMANN-LA ROCHE AG, 4002 Basel (CH)
(72) Inventor: Mergens, William Joseph, West Caldwell, NJ 07006 (US); Raymond, Joseph Edward, Newton, NJ 07860 (US)
(74) Representative: Cottong, Norbert A.

(56) References cited:
- EP-A- 0 363 733
- EP-A- 0 416 773
- US-A- 4 486 435
- US-A- 4 489 026

## Description

The invention relates to micronutrient compositions and to methods of preparing such micronutrient compositions. More particularly, the invention concerns substantially dry micronutrient compositions, aqueous suspensions containing micronutrients and a method for producing the compositions and aqueous suspensions.

Within the class of vitamins, several have recommended daily allowances (RDA) which are relatively low and are thus conveniently expressed in micrograms per day (µg/day). Such vitamins are known as micronutrients. Examples of micronutrients are vitamin B₁₂, which has an RDA of 1.5 to 12.0 µg/day; folic acid, with an RDA of 100 to 800 µg/day; and biotin, with an RDA of 75 to 300 µg/day. The proper distribution of these micronutrients in tablets or other vehicles of administration like foods is a difficult task unless special steps are taken to reduce the crystal size and serially dilute the active substance prior to or during the manufacture of the end product.

United States Patent No. 5,000,888 to Kilbride et al. discloses spray drying a riboflavin slurry to a granulate. The slurry is composed of about 10-50 parts (by weight) of riboflavin, about 0.5-15 parts of a binder and about 50-75 parts of water, such that the mixture contains about 40-75 weight % water. In an example, the process yielded a final composition containing 94 weight % riboflavin, 5 weight % binder and 1 weight % water. As an optional feature the spray drying can be effected in the presence of a small quantity of an absorbant, e.g. silicon dioxide, magnesium carbonate or di- or tricalcium phosphate.

Kilbride (U.S. Patent No. 4,994,458) teaches a riboflavin composition made via a rotary fluidized bed granulator. The product of the process comprises about 75-99.5 weight % riboflavin, and about 0.5-25 weight % binder, e.g. starch, cellulose, dextrin or gelatin.

Schmidt et al. (U.S. Patent No. 4,533,674) teach a process for preparing a powder containing at least 90 weight % ascorbic acid, a maximum of 9 weight % binder, 0.2-2 weight % absorbent, e.g. silicon dioxide, and 0.2-5 weight % lubricant.

Ono et al. (EP 416,773) describe a vitamin B₁₂ composition in which the vitamin is dispersed in a mixture of starch and dextrin, e.g. maltodextrin. The dextrin and starch are mixed together, and an aqueous solution of vitamin B₁₂ is added to form a slurry, which is then spray dried to form the composition. The vitamin B₁₂ is present in the dried composition at about 0.05-1.0 weight % based on the total weight of vitamin, dextrin and starch in the composition.

Cannalonga et al. (U.S. Patent No. 3,914,430) teach a spray drying technique for preparing agglomerated powders containing vitamins and hydrolysed gelatin. The technique involves the introduction of an absorbent material, e.g. silicon dioxide or dicalcium phosphate, into the spray drying chamber with an emulsion of water, vitamin and hydrolyzed gelatin. The absorbent particles are coated onto the emulsion ingredients to form the agglomerates. Examples of resulting products include vitamin E encapsulated in hydrolyzed gelatin, vitamin A encapsulated in hydrolyzed gelatin, vitamin A encapsulated in gum acacia, vitamin D encapsulated in hydrolyzed gelatin and riboflavin encapsulated in maltrin. When dl-α-tocopherol acetate is used as the vitamin the resulting powders have a bulk density in the range of from 25 to 32 lbs/ft³.

Cavalli et al. (U.S. Patent No. 3,396,226) teach dry-mixed compositions containing up to about 80 weight % ascorbic acid, up to about 50 weight % microcrystalline cellulose and/or a corn starch, and a minor amount of a lubricant, e.g. calcium stearate.

Stoyle et al. (U.S. Patent No. 3,293,132) disclose compositions containing 75-95 weight % ascorbic acid, 5-25 weight % carbohydrate, e.g. a sugar or starch, and 0.5-7 weight % binder, e.g. gelatin. The compositions are prepared by dissolving the carbohydrate and binder in water, adding the ascorbic acid to form a slurry having 40-60 weight % solids, and spray drying the homogenized slurry to form a powder. To form tablets from this powder a lubricant, e.g. magnesium stearate, and other ingredients, e.g. silicon dioxide, may be added prior to compression.

Schmidt et al. (WO 85/01877) teach compositions containing a water-soluble vitamin, e.g. ascorbic acid, a salt thereof, folic acid or biotin, a binder, e.g. gelatin or a cellulose, a lubricant, e.g. stearic acid, and preferably also an adsorbent, e.g. silicon diode. Other excipients may also be present e.g. sugars, starch, etc. The compositions are prepared by spray drying an aqueous slurry of a water-soluble vitamin and a binder, in the presence of a lubricant and preferably also an adsorbent.

Schmidt et al. (US Patent No. 4,486,435) indicate in respect of background prior art inter alia that combinations of a medicament and a water-insoluble carrier, e.g. calcium sulphate or dicalcium phosphate, can be spray-dried in the presence of a retarding agent to obtain solid, sustained release pharmaceutical preparations (US Patent No. 3,632,739). Vitamin-containing aqueous colloid beads have been prepared as described in US Patent No. 3,445,563 by spraying droplets of an aqueous gelatin-vitamin mixture into a cooling tower and collecting the resulting powder in admixture with a water-absorbing powder, e.g. silica gel, at the base, and subsequently separating the latter powder therefrom. In this way the vitamin becomes coated with the gelatin, whereby such vitamin powders can be improved in their free-flowing properties by spray-drying said droplets which also contain a water-insoluble carrier in the presence of hydrophilic silica. The invention of Schmidt et al. themselves resides in producing substantially dry, non-agglomerated vitamin-containing powders by spray-drying a solution, dispersion or emulsion of a fat- or water-soluble vitamin. e.g. vitamin B₂ (riboflavin) or B₁₂, in water, also containing an encapsulating agent, e.g. maltodextrin, gelatin, a water-soluble or pregelatinized starch or a cellulose derivative, and a water-insoluble carrier, e.g. an alkaline earth metal sulphate or phosphate, to a powder which is then dried in the presence of a dry absorbent material such as hydrophobic silica. The so-produced powders, with a moisture content of about 1-5 % by weight, contain the vitamin, encapsulating agent and water-insoluble carrier in amounts of about 45 - 60, 10-60 and 2 - 18% by weight, respectively, and the hydrophobic silica content amounts to about 0.2 - 2 % by weight. Accordingly, the vitamin (riboflavin being particularly emphasized) constitutes about half or considerably more than half of the total weight, in contrast to the carrier, which contributes less than one fifth of the weight, even as low as one fiftieth.

The micronutrient compositions of the present invention comprise a micronutrient in an amount up to 15% by weight, a binder in an amount from 7 % to 10 % by weight and a densifying agent in an amount from 75 % to 92 % by weight and having a particle size of from 420 microns (40 mesh) to 44 microns (325 mesh), and have a tapped density of from 0.7 g/cm³ to 1.2 g/cm³ and are dry to the extent that their moisture content is in the range of 1 to 5 weight percent,whereby the densifying agent acts as a core to which the micronutrient becomes adhered with the help of the binder.

The compositions of the invention have direct blendability, excellent flow properties, high densities, and excellent color and chemical stability, making them very suitable for use as additives for foodstuffs and in tablets.

The micronutrient compositions of the present invention feature a core of densifying agent substantially enclosed in a coating of micronutrient and binder. The binder adheres the micronutrient around the central core of densifying agent. In featuring such a laminated structure, the particles of the composition are relatively uniform and the micronutrient on the exterior of the particles is readily accessible. Accordingly, powder formed by the particles of the composition are substantially homogenous and of suitable density to be formed into tablets.

The micronutrient compositions according to the invention are substantially dry to the extent that their moisture content is in the range of 1 weight percent to 5 weight percent. As a result of this substantial dryness they feature inter alia excellent free-flowing properties.

Typical micronutrients suitable for the compositions of the invention are vitamin B₁₂, folic acid, biotin and nutritionally useful derivatives thereof. The micronutrient is present in the composition generally in amounts of up to 15% by weight of the total composition. Preferably, vitamin B₁₂ or biotin is present in an amount from 0.5% to 5% by weight of the total composition, most preferably in an amount of about 1% by weight. For folic acid, the preferred amount is in the range of 5 to 15 weight %, most preferably about 10 weight %.

Species of vitamin B₁₂ suitable for the invention include cyanocobalamin, cyanocobalamin hydrate and indeed any nutritionally useful members of the family of natural and semisynthetic cobalamins. Folic acid and biotin may also be present as such or as any nutritionally useful derivative thereof.

The term "micronutrient" as used in this specification may designate a single micronutrient compound, such as cyanocobalamin, folic acid or biotin itself, or two or more micronutrient compounds. The percentage weight data are accordingly to be understood to relate to the single micronutrient or to the total of two or more micronutrient compounds present, as the case may be. Analogous considerations apply to the terms "binder" and "densifying agent".

The compositions of the invention contain a binder which is present in an amount from 7% to 10% by weight of the total composition. Suitable binders include, for example, those materials which are usually polymeric in nature, which exhibit cohesive properties and which are capable of adhering the active material (micronutrient) to the densifier, and in addition can protect the active material from chemical hydrolysis, oxidation and any other adverse effect which would normally lead to its deterioration. Preferred binders are maltodextrins, gelatins, starches, and celluloses, with maltodextrins being the most preferred binders. Preferably the binder is present in an amount from 7% to 8% by weight of the total composition.

The compositions of the invention also contain a densifying agent in an amount from 75% to 92% by weight of the total composition, preferably, from 80% to 90% by weight. Typical densifying agents include, for example, alkaline earth sulfates, alkali carbonates and alkaline earth phosphates. Preferred densifying agents are calcium sulfate, barium sulfate and dicalcium phosphate dihydrate. As indicated hereinabove the densifying agents used in the preparation of the compositions have a particle size of from 420 microns (40 mesh) to 44 microns (325 mesh).

As well as a micronutrient, a binder and a densifying agent the micronutrient compositions of the invention also preferably contain silicon dioxide. When present, the silicon dioxide is preferably present in an amount in the range from 0.5 to 1.5% by weight of the total composition.

The compositions of the invention have tapped densities of from 0.7g/cm³ to 1.2g/cm³. Preferably, the tapped density of the composition is from 0.8g/cm³ to 1.1g/cm³. The indicated "tapped" densities are those as determined in the commercially available Van der Kamp tap density tester. The procedure involves placing a 100 ml graduated cylinder, which has been charged with a 50 g portion of the pulverous or granulated test material, into the tap density tester and applying 3 taps (movements) per second at a travel distance of 1.9 cm per tap. After 100 taps the volume of the test material in the graduated cylinder is measured. The procedure is repeated several times using a fresh 50 g portion of test material each time, and the average of the volumes measured taken for the final tapped density calculation.

Another aspect of the invention is an aqueous suspension comprising water in an amount at least 35% by weight and solids in an amount up to 65% by weight, whereby the solids comprise a dissolved or dispersed micronutrient in an amount up to 15% by weight of the total solids, a binder in an amount from 7% to 10% by weight and a water-insoluble densifying agent in an amount from 75% to 92% by weight. Typically, the suspensions according to the present invention have a density from 1.4 g/cm³ to 1.6 g/cm³. For example, the suspensions of the invention, at room temperature (23°C) and with a 65% solids content have densities of approximately 1.57 g/cm³ (1% biotin), 1.57 g/cm³ (1% cyanocobalamin) or 1.44 g/cm³ (10% folic acid). In contrast, conventional spray dried feed solutions have densities in the range of about 0.98 to about 1.2 g/cm³. The densities of some typical conventional spray dried feed solutions are presented in the following table:

**TABLE**

| Product Description | Density of Feed Solution (g/cm³) | Temp., °C |
|---|---|---|
| Dry Vitamin E 50% - Emulsion (oil + H₂O/gelatin) | 1.047-1.050 | 55-60 |
| Rocoat® Thiamine Mononitrate 33 1/3% -Suspension in Wax | 1.010 | 75 |
| Rocoat® Riboflavin 33 1/3% -Suspension in Wax | 1.075 | 75 |
| Rocoat® Pyridoxine HC1 33 1/3% -Suspension in Wax | 1.000 | 75 |
| Rocoat® Niacinamide 33 1/3% -Suspension in Wax | 0.980 | 75 |
| Vitamin D₃ 2000 SD - Emulsion | 1.102 | 60 |
| Sodium Sulfadimethoxine - Solution | 1.190 - 1.200 | 25 |

The aqueous suspensions of the invention can be spray dried to the substantially dry micronutrient compositions according to the invention.

Another aspect of the invention concerns the process used in producing the substantially dry micronutrient compositions of the invention. This process is characterized by the steps of a) dissolving or dispersing appropriate amounts of the micronutrient and the binder in water, b) suspending an appropriate amount of the densifying agent in the solution or suspension obtained from step a) and c) spray drying the suspension obtained from step b) to produce the desired substantially dry micronutrient composition.

The aqueous suspension of the present invention is produced according to the steps a) and b) as indicated hereinabove, using appropriate amount of solids and water. A suspension is formed in step b) because the densifying agent is substantially insoluble in the solution or dispersion formed in step a).

The process of the invention is advantageous in that the regulation of the particle size of the so obtained substantially dry micronutrient composition is automatically regulated, i.e. regulated without the need to manipulate the spray drying conditions of the process step c). This advantage is attributable to the use of a unique combination of micronutrient and binder, and to the size selection of the densifying agent (particle size of from 420 microns ( 40 mesh) to 44 microns (325 mesh)) which is substantially insoluble in the solution or suspension of micronutrient and binder in water obtained in the process step a). In the process the densifying agent acts as a core to which the micronutrient becomes adhered with the help of the binder. The result is a particle of slightly larger size than the particle of densifying agent itself.

Preferably, the inlet temperature of the spray-dryer is between 180°C and 200°C, and the outlet temperature is between 85°C and 110°C, with the overall change in temperature between inlet and outlet being approximately 70°C to 115°C. Since the process of spray drying is well known in the art, the specific spray drying process conditions needed for the invention are not critical and thus can be determined by one having skill in the art. Thus, amongst other factors, the spray drying is preferably effected in the presence of silicon dioxide, and in this case the amount of silicon dioxide used is preferably such as to yield a substantially dry micronutrient composition containing silicon dioxide in an amount from about 0.5 to about 1.5% by weight of the total composition.

In a particularly preferred embodiment of the process of the present invention vitamin B₁₂, maltodextrin and calcium sulfate are used as the three solid components in a weight ratio of approximately 1:10:89 respectively, and the suspension obtained from step b) is spray dried in the presence of silicon dioxide such that the final product also contains silicon dioxide to the extent of about 0.5 to 1.5% by weight.

The following examples illustrate the present invention:

### EXAMPLES

In general, the procedure for preparing the compositions of the invention entails charging a suitable vessel with the formulation amount of distilled or deionized water at approximately 45-60°C. With continuous agitation, the formulation amount of binder is added and mixed until dissolved. The micronutrient then is added to the solution and mixed until completely solubilized or dispersed. To the solution is added the formulation amount of the insoluble densifying agent and the slurry is mixed until homogeneous flow properties are obtained. The slurry then can then be spray dried according to conventional methods.

### EXAMPLE 1

### 1% Vitamin B₁₂ Formulation

In a 6 liter vessel, 1500 ml of water were heated to approximately 70°C. To the water were added 200 grams of Maltrin MD 040 (maltodextrin) with continuous mixing until dissolved. Then 25.0 grams of vitamin B₁₂ hydrate (88.1% B₁₂) were added to the solution and the solution mixed until the vitamin was dissolved. With continuous mixing, calcium sulfate (1775 grams) was slowly added to the vitamin solution until a uniform suspension resulted. The suspension was then gravity fed into a Niro 40 Utility spray dryer having an inlet temperature of 185°C, an outlet temperature of 100°C, and a wheel speed of 15,000 rpm, with 1.5 weight percent silicon dioxide (Syloid® 74) being simultaneously fed into the dryer.

The tapped density of the resulting substantially dry composition was 0.88g/cm³ (54.9 lb/ft³).

### EXAMPLE 2

### 10% Folic Acid Formulation

In a 6 liter vessel, 1000 ml of water were heated to approximately 60°C. To the water were added 200 grams of Maltrin MD 040 (maltodextrin) with continuous mixing until dissolved. Then 240 grams of folic acid (92%) were added to the solution and the solution mixed until the folic acid was dissolved. With continuous mixing, calcium sulfate (1560 grams) was slowly added, along with an additional 1000 ml of water, to the folic acid solution until a uniform suspension resulted. The suspension was then gravity fed into a Niro 40 Utility spray dryer having an inlet temperature of 180°C, an outlet temperature of 90°C-100°C, and a wheel speed of 20,000 rpm, with 1.5 weight percent silicon dioxide (Syloid® 74) being simultaneously fed into the dryer.

The tapped density of the resulting substantially dry composition was 0.89 g/cm³ (55.6 lb/ft³).

### EXAMPLE 3

### 1% Biotin Formulation

In a 6 liter vessel, 1500 ml of water were heated to approximately 70°C. To the water were added 200 grams of Maltrin MD 404 (maltodextrin) with continuous mixing until dissolved. Then 22.0 grams of Biotin USP, Food Chemicals Codex (FCC), were added to the solution and the solution mixed until the biotin had dispersed. With continuous mixing, calcium sulfate (1778 grams) was slowly added to the biotin solution until a uniform suspension resulted. The suspension then was gravity fed into a Niro 40 Utility spray dryer having an inlet temperature of 190°C-200°C, an outlet temperature of 100°C-110°C, and a wheel speed of 15,000 rpm, with 1.5 weight percent silicon dioxide (Syloid® 74) being simultaneously fed into the dryer.

The loose bulk density of the resulting substantially dry composition was 86.5 g/100 cm³ (54 lb/ft³), and the tapped density was 103.0g/100 cm³ (64.3 lb/ft³).

### EXAMPLE 4

### 1% Biotin Formulation

In a 6 liter vessel, 1500 ml of water were heated to approximately 70°C. To the water were added 200 grams of Maltrin MD 404 (maltodextrin) with continuous mixing until dissolved. Then 22.0 grams of Biotin USP, FCC, was added to the solution and the solution mixed until the biotin had dispersed. With continuous mixing, dicalcium phosphate dihydrate (1778 grams) was slowly added to the biotin solution until a uniform suspension resulted. The suspension was then gravity fed into a Niro 40 Utility spray dryer having an inlet temperature of 190°C-200°C, an outlet temperature of 100°C-110°C, and a wheel speed of 15,000 rpm, with 1.5 weight percent silicon dioxide (Syloid® 74) being simultaneously fed into the dryer.

The loose bulk density of the resulting substantially dry composition was 76.6 g/100 cm³ (47.8 lb/ft³), and the tapped density was 87.9g/100 cm³ (54.9 lb/ft³).

## Claims

1. A micronutrient composition comprising a micronutrient in an amount up to 15% by weight, a binder in an amount from 7 % to 10 % by weight and a densifying agent in an amount from 75% to 92% by weight and having a particle size of from 420 microns (40 mesh) to 44 microns (325 mesh), said composition having a tapped density of from 0.7 g/cm³ to 1.2 g/cm³ and being dry to the extent that its moisture content is in the range of 1 to 5 weight percent, whereby the densifying agent acts as a core to which the micronutrient becomes adhered with the help of the binder.

2. A composition according to claim 1, wherein the micronutrient is vitamin B₁₂, folic acid or biotin.

3. A composition according to claim 1 or 2, wherein the binder is a maltodextrin, gelatin, cellulose or starch.

4. A composition according to any preceding claim, wherein the densifying agent is an alkaline earth sulfate, an alkali carbonate or an alkaline earth phosphate.

5. A composition according to claim 4, wherein the densifying agent is calcium sulfate, barium sulfate or dicalcium phosphate dihydrate.

6. A composition according to any preceding claim, wherein the binder is present in an amount from 7% to 8% by weight, and the densifying agent is present in an amount from 80% to 90% by weight, of the total composition.

7. A composition according to any preceding claim, comprising, in addition to the micronutrient, binder and densifying agent, silicon dioxide.

8. A composition according to claim 7, wherein the silicon dioxide is present in an amount in the range from 0.5 to 1.5% by weight of the total composition.

9. An aqueous suspension comprising water in an amount at least 35% by weight and solids in an amount up to 65% by weight, whereby the solids comprise a dissolved or dispersed micronutrient in an amount up to 15% by weight of total solids, a binder in an amount from 7% to 10% by weight and a water-insoluble densifying agent in an amount from 75% to 92% by weight and having a particle size of from 420 microns (40 mesh) to 44 microns (325 mesh).

10. A process for producing a micronutrient composition as defined in any one of claims 1 to 8, characterized by the steps of
a) dissolving or dispersing appropriate amounts of the micronutrient and the binder in water,
b) suspending an appropriate amount of the densifying agent in the solution or dispersion obtained from step a) and
c) spray drying the suspension obtained from step b) to produce the desired composition.

## Patentansprüche

1. Mikronährstoffzusammensetzung mit einer Partikelgrösse von 420µm (40 mesh) bis 44µm (325 mesh) enthaltend einen Nährstoff in einer Menge bis zu 15 Gew.-%, einen Binder in einer Menge von 7-10 Gew.-% und ein Dichtungsmittel in einer Menge von 75-92 Gew.-%, wobei die Zusammensetzung eine Stampfdichte von 0,7-1,2 g/cm³ aufweist und derart trocken ist, dass ihr Feuchtigkeitsgehalt im Bereich von 1-5 Gew.-% liegt, wobei das Dichtungsmittel als Kern dient, an welchem der Nährstoff mit Hilfe des Binders anhaftet.

2. Zusammensetzung nach Anspruch 1, wobei der Nährstoff Vitamin B₁₂, Folsäure oder Biotin ist.

3. Zusammensetzung nach Anspruch 1 oder 2, wobei der Binder Maltodextrin, Gelatine, Zellulose oder Stärke ist.

4. Zusammensetzung nach einem der vorigen Ansprüche, wobei das Dichtungsmittel ein Erdalkalisulfat, ein Alkalicarbonat oder ein Erdalkaliphosphat ist.

5. Zusammensetzung nach Anspruch 4, wobei das Dichtungsmittel Calciumsulfat, Bariumsulfat oder Dicalciumphophatdihydrat ist.

6. Zusammensetzung nach einem der vorigen Ansprüche, wobei der Binder in einer Menge von 7-8 Gew.-% und das Dichtungsmittel in einer Menge von 80-90 Gew.-% der Gesamtzusammensetzung anwesend ist.

7. Zusammensetzung nach einem der vorigen Ansprüche enthaltend Siliziumdioxid zusätzlich zu dem Nährstoff, Binder und Dichtungsmittel.

8. Zusammensetzung nach Anspruch 7, wobei Siliziumdioxid in einer Menge von 0,5-1,5 Gew.-% der Gesamtzusammensetzung anwesend ist.

9. Wässrige Suspension enthaltend Wasser in einer Menge von wenigstens 35 Gew.-% und Feststoffe in einer Menge von bis zu 65 Gew.-%, wobei die Feststoffe einen gelösten oder dispergierten Mikronährstoff in einer Menge von bis zu 15 Gew.-% der gesamten Feststoffe, einen Binder in einer Menge von 7-10 Gew.-% und ein wasserunlösliches Dichtungsmittel in einer Menge von 75-92 Gew.-% enthalten und eine Partikelgrösse von 420µm (40 mesh) bis 44µm (325mesh) aufweisen.

10. Verfahren zur Herstellung einer Mikronährstoffzusammensetzung nach einem der Ansprüche 1-8 gekennzeichnet durch die Verfahrensstufen
a) Lösen oder Dispergieren entsprechender Mengen an Nährstoff und Binder in Wasser,
b) Suspendieren einer entsprechenden Menge an Dichtungsmittel in die aus Verfahrensstufe (a) erhaltenen Lösung oder Dispersion und
c) Sprühtrocknen der aus Verfahrensstufe b) erhaltenen Suspension, um die gewünschte Zusammensetzung zu erhalten.

## Revendications

1. Composition de micronutriments comprenant un micronutriment en une quantité pouvant atteindre 15 % en poids, un liant en une quantité de 7 % à 10 % en poids et un agent densifiant en une quantité de 75 % à 92 % en poids et ayant un taille de particule de 420 micromètres ( maille 40) à 44 micromètres ( maille 325) , ladite composition ayant une densité tassée de 0,7 g/cm³ à 1,2 g/ cm³ et étant séchée de telle façon que sa teneur en humidité soit comprise dans l'intervalle de 1 à 5 pourcent en poids, où l'agent densifiant joue le rôle d'un coeur où le micronutriment est collé à l'aide du liant.

2. Composition selon la revendication 1, où le micronutriment est la vitamine B₁₂, l'acide folique ou la biotine.

3. Composition selon la revendication 1 ou 2, où le liant est une maltodextrine, une gélatine, une cellulose ou un amidon.

4. Composition selon l'une quelconque des revendications précédentes, où l'agent densifiant est un sulfate de métal alcalino-terreux, un carbonate de métal alcalin ou un phosphate de métal alcalinoterreux.

5. Composition selon la revendication 4, où l'agent densifiant est du sulfate de calcium, du sulfate de baryum ou du phosphate dicalcique dihydrate.

6. Composition selon l'une quelconque des revendications précédentes, où le liant est présent en une quantité de 7 % à 8 % en poids , et l'agent densifiant est présent en une quantité de 80 % à 90 % en poids, de la composition totale.

7. Composition selon l'une quelconque des revendications précédentes, comprenant en plus du micronutriment, du liant et de l'agent densifiant, du dioxyde de silicium.

8. Composition selon la revendication 7, où le dioxyde de silicium est présent en une quantité comprise dans l'intervalle de 0,5 à 1,5 % en poids de la composition totale.

9. Suspension aqueuse comprenant de l'eau en une quantité d'au moins 35 % en poids et des solides en une quantité pouvant atteindre 65 % en poids, où les solides comprennent un micronutriment dissout ou dispersé en une quantité pouvant atteindre 15 % en poids du total des solides, un liant en une quantité de 7 % à 10 % en poids et un agent densifiant insoluble dans l'eau en une quantité de 75 % à 92 % en poids et ayant une taille de particule de 420 micromètres ( maille 40) à 44 micromètres ( maille 325).

10. Procédé de production d'une composition de micronutriment selon l'une quelconque des revendications 1 à 8, caractérisé par les étapes de
a) dissoudre ou disperser des quantités appropriées du micronutriment et de liant dans l'eau,
b)mettre en suspension une quantité appropriée de l'agent densifiant dans la solution ou la dispersion obtenue à l'étape a) et
c) sécher par atomisation la suspension obtenue à l'étape b) pour produire la composition souhaitée.
